Europäisches Patentamt

**European Patent Office**

Office européen des brevets

(19)

(11) Veröffentlichungsnummer: **0 281 608**
**B1**

(12) **EUROPÄISCHE PATENTSCHRIFT**

(45) Veröffentlichungstag der Patentschrift: **11.07.90**

(51) Int. Cl.⁵: **C 07 D 209/14, A 61 K 31/44**

(21) Anmeldenummer: **87906082.0**

(22) Anmeldetag: **12.09.87**

(86) Internationale Anmeldenummer:
**PCT/EP87/00518**

(87) Internationale Veröffentlichungsnummer:
**WO 88/01998 24.03.88 Gazette 88/07**

(54) **HYDROXYINDOLDERIVAT.**

(30) Priorität: **16.09.86 US 907909**

(43) Veröffentlichungstag der Anmeldung:
**14.09.88 Patentblatt 88/37**

(45) Bekanntmachung des Hinweises auf die
Patenterteilung:
**11.07.90 Patentblatt 90/28**

(84) Benannte Vertragsstaaten:
**AT BE CH DE FR GB IT LI NL SE**

(56) Entgegenhaltungen:
**EP-A-0 007 399**

(73) Patentinhaber: **MERCK PATENT GESELLSCHAFT
MIT BESCHRÄNKTER HAFTUNG**
**Frankfurter Strasse 250 Postfach 4119**
**D-6100 Darmstadt (DE)**

(72) Erfinder: **HAUSBERG, Hans-Heinrich**
**Odenwaldstrasse 30**
**D-6105 Ober-Ramstadt (DE)**
Erfinder: **BÖTTCHER, Henning**
**Soderstrasse 95**
**D-6100 Darmstadt (DE)**
Erfinder: **SEYFRIED, Christoph**
**Mathildenstrasse 6**
**D-6104 Seeheim-Jugenheim (DE)**
Erfinder: **BERGMANN, Rolf**
**Birkenhag 36**
**D-6101 Reichelsheim (DE)**

Courier Press, Leamington Spa, England.

**Beschreibung**

Die Erfindung betrifft das neue 3 - [4 - (4 - Phenyl - 1,2,3,6 - tetrahydro - 1 - pyridyl) - butyl] - 5 - hydroxy-indol - methansulfonat (I).

Die zugrundeliegende Base (II) sowie das entsprechende Hydrochlorid (III) sind in der DE—OS 29 10 367 beschrieben.

Es wurde gefunden, daß I erheblich günstigere pharmakokinetische Eigenschaften besitzt als III. So führen z.B. bereits 100 mg/kg I oral an Cynomolgus-Affen zu einer sedierenden Reaktion, während dieser Effekt mit III erst bei Dosen oberhalb 500 mg/kg erzielt werden kann. Außerdem ist I in Wasser wesentlich besser löslich (0,921 g in 100 ml) als III (0,035 g in 100 ml).

Gegenstand der Erfindung ist dementsprechend die neue Verbindung I sowie ein Verfahren zu ihrer Herstellung, das darin besteht, daß man die Base II mit Methansulfonsäure umsetzt.

Diese Umsetzung erfolgt zweckmäßig in einem inerten Lösungsmittel, z.B. Methanol oder Ethanol, bei Temperaturen zwischen 0 und 80°, vorzugsweise zwischen 15 und 30°. Vorzugsweise verwendet man stöchiometrische Mengen der Ausgangstoffe.

Die Verbindung I kann zur Herstellung pharmazeutischer Zubereitungen verwendet werden, insbesondere auf nichtchemischen Wege. Hierbei kann sie zusammen mit mindestens einem festen, flüssigen oder halb-flüssigen Träger- oder Hilfsstoff und gebenenfalls in Kombination mit einem oder mehreren weiteren Wirkstoff(en) in eine geeignete Dosierungsform gebracht werden.

Gegenstand der Erfindung sind ferner pharmazeutische Zubereitungen, enthaltend die Verbindung I. Diese Zubereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topische Applikation eignen und mit der neuen Verbindung nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neue Verbindung kann auch lyophilisiert werden, und die erhaltenen Lyophilisate können z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindung I kann an Menschen oder Tiere, insbesondere Säugetiere wie Affen, Hunde, Katzen, Ratten oder Mäuse verabreicht und bei der therapeutischen Behandlung des menschlichen oder tierischen Körpers und bei der Bekämpfung von Krankheiten verwendet werden, insbesondere bei der Therapie von Parkinsonismus, von extrapyramidalen Störungen bei der Neuroleptikatherapie, von Depressionen und/oder Psychosen und von Nebenwirkungen bei der Behandlung der Hypertonie (z.B. mit α-Methyldopa). Ferner kann die Verbindung I in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z.B. Migräne), insbesondere in der Geriatrie ähnlich wie gewisse Ergot-Alkaloide, sowie auch zur Blutdrucksenkung.

Dabie wird die Verbindung I in der Regel in Analogie zu bekannten, im Handel befindlichen Präparaten (z.B. Bromocriptin, Dihydroergocornin) verabreicht, vorzugsweise in Dosierungen zwischen etwa 0,05 und 5 mg, insbesondere zwischen 0,2 und 2 mg pro Dosierungseinheit. Die tägliche Dosierung liegt vorzugsweise zwishen etwa 0,001 und 0,1 mg/kg Körpergewicht. Die spezielle Dosis für jeden bestimmten Patienten hängt jedoch von de verschiedensten Faktoren ab, beispielsweise vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Herstellungsbeispiel

Zu einer Suspension von 23 g 3 - [4 - (4 - Phenyl - 1,2,3,6 - tetrahydropyridyl) - butyl] - 5 - hydroxyindol (II) in 340 ml Ethanol gibt man bei 15°C eine Lösung von 7 g Methansulfonsäure in 40 ml Ethanol, erwärmt kurz auf 30°C, kühlt auf 15°C und filtriert das erhaltene 3 - [4 - (4 - Phenyl - 1,2,3,6 - tetrahydro - 1 - pyridyl) - butyl] - 5 - hydroxyindol - methansulfonat (I) ab. F. 176°C.

Die nachstehenden Beispiele betreffen pharmazeutische Zubereitungen, die die Verbindung I enthalten:

Beispiel A: Tabletten

Ein Gemisch von 1 kg I, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 1 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt 10 kg I in üblicher Weise in Hartgelatinekapseln, so daß jede Kapsel 1 mg Wirkstoff enthält.

Beispiel D: Ampullen

Eine Lösung von 1 kg I in 30 I zweifach destilliertem Wasser wird steril filtriert, in Ampullen abgefüllt, unter sterilen Bedingungen lyophilisiert und steril verschlossen. Jede Ampulle enthält 0,5 mg Wirkstoff.

**Patentantsprüche**

1. 3 - [4 - (4 - Phenyl - 1,2,3,6 - tetrahydro - 1 - pyridyl) - butyl] - 5 - hydroxy - indol - methansulfonat (I).

2. Verfahren zur Herstellung von Verbindung I nach Anspruch 1, dadurch gekennzeichnet, daß man 3 - [4 - (4 - Phenyl - 1,2,3,6 - tetrahydro - 1 - pyridyl) - butyl] - 5 - hydroxy - indol mit Methansulfonsäure umsetzt.

3. Verfahren zur Herstellung pharmazeutischer Zubereitungen, dadurch gekennzeichnet, daß man die Verbindung I nach Anspruch 1 zusammen mit mindestens einem festen, flüssigen oder halbflüssigen Träger- oder Hilfsstoff und gegebenenfalls in Kombination mit einem oder mehreren weitern Wirkstoffen in eine geeignete Dosierungsform bringt.

4. Pharmazeutische Zubereitung, gekennzeichnet, durch einen Gehalt an der Verbindung I nach Anspruch 1.

5. Verbindung I nach Anspruch 1, zur Bekämpfung von Krankheiten.

6. Verwendung von Verbindung I nach Anspruch 1 zur Herstellung von Arnzeimitteln.

**Revendications**

1. Le méthane-sulfonate du 3 - [4 - (4 - phényl - 1,2,3,6 - tétrahydro - 1 - pyridyl) - butyl] - 5 - hydroxy indole (I).

2. Procédé de préparation du composé I de la revendication 1, caractérisé en ce que l'on fait réagir le 3 - [4 - (4 - phényl - 1,2,3,6 - tétrahydro - 1 - pyridyl) - butyl] - 5 - hydroxy - indole avec l'acide méthane-sulfonique.

3. Procédé de préparation de compositions pharmaceutique, caractérisé en ce que l'on met le composé I de la revendication 1, accompagné d'au moins un véhicule ou produit auxiliaire solide, liquide ou semiliquide et le cas échéant en combinaison avec une ou plusieurs autres substances actives, sous une forme de dosage appropriée.

4. Composition pharmaceutique caractérisé en ce qu'elle contient le composé I de la revendication 1.

5. Le composé I de la revendication 1, pour le traitement de maladies.

6. Utilization du composé I de la revendication 1, pour la préparation de médicaments.

**Claims**

1. 3 - [4 - (4 - Phenyl - 1,2,3,6 - tetrahydro - 1 - pyridyl) - butyl] - 5 - hydroxy - indole methanesulfonate (I).

2. Process for the preparation of the compound I according to claim 1, characterized in that 3 - [4 - (4 - phenyl - 1,2,3,6 - tetrahydro - 1 - pyridyl) - butyl] - 5 - hydroxy - indole is reacted with methanesulfonic acid.

3. Process for the preparation of pharmaceutical formulations, characterized in that the compound I according to Claim 1, is brought into a suitable dosage form together with at least one solid, liquid or semi-liquid excipient or auxiliary and if appropriate in combination with one or more other active compounds.

4. Pharmaceutical formulation, characterized in that it contains the compound I according to Claim 1.

5. Compound I according to Claim 1 for combating diseases.

6. Use of the compound I according to Claim 1 for the preparation of medicaments.